# EUROPEAN PATENT SPECIFICATION

(11) **EP 1 753 309 B1**
(45) Date of publication and mention of the grant of the patent: **02.06.2010**
(21) Application number: 05747335.7
(22) Date of filing: 27.05.2005
(51) Int. Cl.: A23L 1/305, A23G 3/00, A61K 31/00

(54) **FOOD COMPOSITIONS CONTAINING CREATINE**
KREATIN ENTHALTENDE NAHRUNGSMITTELZUSAMMENSETZUNGEN
COMPOSITIONS ALIMENTAIRES CONTENANT DE LA CREATINE

(30) Priority: 28.05.2004 GB 0411985
(43) Date of publication of application: 21.02.2007
(73) Proprietor: Howard Foundation Holdings Limited, Great Shelford Cambridgeshire CB2 5JY (GB)
(72) Inventor: HARRIS, Roger Charles, Newmarket, Suffolk CB8 8HD (GB); HOWARD, Alan Norman, Great Shelford, Cambridge CB2 5JY (GB)
(74) Representative: Lipscombe, Martin John
(86) International application number: PCT/GB2005/002127
(87) International publication number: WO 2005/115175

(56) References cited:
- WO-A-00/74500
- WO-A-01/33976
- WO-A-01/70238
- WO-A-98/53704
- US-A1- 2004 137 112
- MCCARTY M F: "Nutraceutical resources for diabetes prevention - an update" MEDICAL HYPOTHESES, EDEN PRESS, PENRITH, US, vol. 64, no. 1, 2005, pages 151-158, XP004729055 ISSN: 0306-9877

## Description

### Field of the Invention

This invention relates to food compositions for human consumption comprising creatine and to a method of providing such compositions. In particular the invention relates to snack bars comprising creatine, especially snack bars having a low glycemic index.

### Background to the Invention

In the last few years there has been considerable interest among athletes in creatine, which occurs abundantly in skeletal muscle. Creatine plays a pivotal role in the regulation and homeostasis of skeletal muscle energy metabolism and it is now generally accepted that the maintenance of phospho-creatine availability is important to the continuation of muscle force production. Creatine may also be involved in other processes concerned with protein synthesis and hypertrophy of muscle fibres during training. Although creatine synthesis occurs in the liver, kidney and pancreas it has been known for sometime that the oral ingestion of creatine will add to the whole body creatine pool, and it has been shown that the ingestion of 20 to 30g creatine monohydrate (Cr.H₂O) per day for several days can lead to a greater than 20% increase in human skeletal muscle total creatine content. Thus, WO94/02127 discloses the administration of creatine monohydrate in amounts of at least 15g (or 0.2-0.4 g/kg body weight) per day, for at least 2 days, for increasing muscular strength.

In fact, it was subsequently found that after several days of supplementation (20g per day) with creatine monohydrate in order to attain initial elevation of the tissue stores, thereafter it takes no more than 2 to 3g per day to maintain the newly elevated concentration. Supplementation with any bioavailable source of creatine (i.e. creatine supplementation) in an appropriate dose can provide improvements to athletes involved in sustained or intermittent high intensity events, including all events lasting from a few seconds to a few minutes (such as sprinting, swimming, weight-lifting etc). Endurance performance in events lasting longer than about 30 minutes appear less affected by creatine supplementation except where this involves short periods of increased energy output particularly when the local muscle carbohydrate stores have become depleted. Creatine is a normal food component and is not a drug and its use is not contrary to official regulations.

Conventionally, creatine is consumed by athletes in combination with glucose. The co-administration of creatine and glucose has been shown to optimise the uptake of creatine by muscles (Greenhaff et al, US 5,968,900).

Creatine is unstable and can be converted fairly readily into the related compound creatinine. This is significant, because creatinine has no muscle performance-enhancing effects. The rate of conversion of creatine to creatinine is faster at acidic pH than in alkaline conditions. Most drinks or foodstuffs for human consumption are acidic, since alkaline substances possess a disagreeable "soapy" taste. Because of the instability of creatine, creatine-containing drinks are usually made up just prior to consumption, by mixing a flavoured creatine-containing formulation with an appropriate volume of water or other liquid.

WO 98/53704 (Bailey et al) discloses a cereal bar comprising creatine.

WO 01/35953 (Kuhrts) discloses muscle enhancing formulations comprising creatine and an "insulin modulating agent", such as arginine. The formulation is preferably a sustained-release formulation.

US 6,075,031 discloses the use of creatine compounds such as cyclocreatine and creatine phosphate for treating or preventing insulin-related conditions such as hyperglycaemia, insulin-dependent diabetes mellitus (IDDM), impaired glucose tolerance, hyperinsulinaemia, insulin insensitivity and diabetes related diseases.

WO 01/33976 discloses a food composition having a low glycemic index which is useful for treating a patient with Type 2 diabetes and other blood glucose related conditions.

US 2004/0137112 discloses a food composition having a low glycemic index and having a relatively high carbohydrate content.

### Summary of the Invention

In a first aspect the invention provides a solid or semi-solid snack bar for human consumption, comprising creatine suspended in solid form in an edible supporting matrix; the snack bar as a whole having a glycemic index of 55 or below.

The glycemic index is a measure of the ability of a nutrient to raise blood glucose levels after ingestion and is defined as the incremental area under the blood glucose response curve of a 50g carbohydrate portion of a test food expressed as a percent of the response to the same amount of carbohydrate as pure glucose taken by the same subject (The Glucose Revolution. The Authoritative Guide to the Glycemic Index. J.Brand-Miller et al. Marlowe and Co. New York, 1999). Thus, the index compares the effect of a substance on blood glucose levels with the effect caused by glucose itself, utilising in the test equal amounts of carbohydrate. A low glycemic index is defined, for present purposes, as any index of 55 or below. Such low glycemic index substances are especially useful in foodstuffs intended for consumption by diabetics or for those seeking to lose weight. The combination of creatine with carbohydrates with a low glycemic index is contrary to current commercial practice (WO96/183139 Commercial products combining creatine with carbohydrate emphasise the use of those carbohydrates with a *high* glycemic index which stimulate the release of insulin. This is recommended as insulin has been shown to increase the uptake of creatine into muscle at least in the early stages of creatine loading. The present inventors prefer however to avoid such high glycemic index foods.

It will be appreciated that whilst the snack bar as a whole may have a low glycemic index (i.e. a value of 55 or below), some individual components of the bar may have a higher glycemic index, but these will be offset by the presence of other components (preferably present in larger amounts) with a low glycemic index, such that the average glycemic index of the bar as a whole is 55 or below.

A low glycemic index for the bar as a whole can be obtained by using a preponderance of ingredients which either (a) do not contain carbohydrate or which (b) contain carbohydrate with a low glycemic index. Examples of the former include: meat extracts and protein isolates (e.g. caseinates, especially calcium caseinate), soy or whey protein; oils and fats (e.g. vegetable oils such as sunflower oil, olive oil, palm kernel oil; hydrogenated vegetable oils; margarines; butter) - preferably in small amounts to keep calorific value low; artificial sweeteners (such as saccharine, aspartame, acetsulfam K, sucralose); dietary fibre (e.g. barley, bran, oats, chicory); and emulsifiers (e.g. soya lecithin).

Examples of carbohydrates with a low glycemic index (or substances comprising such) include: certain sugars, such as fructose, very high fructose syrups or brown rice syrup; sugar alcohols (such as maltitol, sorbitol and glycerol); and cocoa powder or liquor, comprising low glycemic index sugars, sugar alcohols and/or artificial sweeteners. Cocoa powder or liquor is especially useful for making a chocolate-flavoured coating for the snack bar.

Other ingredients which may conveniently be included in the snack bar include one or more, in any combination, of the following: fresh or dried fruit (especially apples, cherries, oranges, peaches, plums or grapefruit, any of which may comprise pulp, peel, zest or a mixture thereof); vegetables (especially carrots); milk products (including dried whole or reduced fat milk powder, yogurt and cream); oatmeal and durum wheat flours and products derived therefrom; beans (especially butter, kidney, navy or soy beans); lentils and other low glycemic index seeds; dried soy milk; nuts (including peanuts, cashews and hazelnuts) which may be whole, chopped or pasted; raising agent (e.g. sodium bicarbonate and disodium phosphate); binding agents; preservatives (e.g. potassium benzoate, potassium sorbate); and vitamins and minerals.

Preferred vitamins and minerals include: dicalcium phosphate, dipotassium phosphate, magnesium oxide, monocalcium phosphate, ascorbic acid, ferric orthophosphate, vitamin E acetate, niacinamide, zinc oxide, copper gluconate, d-calcium pantothenate, manganese sulfate, vitamin A palmitate, pyridoxine hydrochloride, thiamin mononitrate, riboflavin, sodium molybdate, chromium chloride, folic acid, biotin, sodium selenite, potassium iodide, vitamin K1 (phytonadione), and vitamin B12.

The mineral and trace elements can also be added in any type or form which is suitable for human consumption. It is convenient to provide the calcium and potassium in the form of their gluconates, phosphates or hydrogen phosphates, and magnesium as the oxide or carbonate, chromium as chromium picolinate, selenium as sodium selenite or selenate, and zinc as zinc gluconate. Typically the amounts are:- sodium at 400mg/Kg, calcium at 100mg/Kg, chloride at 600mg/Kg, potassium at 200mg/Kg, magnesium at 75mg/Kg and phosphorus at 50mg/Kg, chromium at 125 g/Kg, selenium at 125 g/Kg and zinc at 15mg/Kg.

The creatine content may be present as crystals or powder, and is distributed within the edible supporting matrix, which may be a viscous liquid or semi-liquid or a solid, typically such that settling of the solid creatine (under the influence of gravity) is inhibited or prevented.

The creatine content of the composition is present as creatine monohydrate. The creatine content of the composition is preferably subjected to a micronization process (e.g. crushing, pulverizing, powdering prior to incorporation into the matrix, so that the resulting composition is not unacceptably "gritty" in texture.

Preferably the creatine will be distributed substantially evenly throughout the supporting matrix (by homogenizing in some manner e.g. by mixing, stirring, blending), which may be accomplished manually and/or mechanically at the time the composition is prepared.

Conveniently the supporting matrix is, or comprises, a recognised foodstuff, such that a composition in accordance with the invention may take the form of an otherwise conventional foodstuff, supplemented with creatine, such that solid creatine becomes suspended in the foodstuff. Examples of foodstuffs which may represent suitable supporting matrices for the composition of the invention include spreadable solids such as fat, butter, margarine and the like. Other convenient supporting matrices are those comprising low glycemic index sugars or other carbohydrates, such as brown rice syrup. Fat, butter or margarine are preferred, as they can also serve to bind the ingredients together.

If desired, the viscosity of the edible matrix and/or the composition as a whole, may be increased by the addition of viscosifiers, gelling agents. Such components are well-known in the foods industry and include, for example, plant-derived polysaccharides, gums such as galactomannans, dextrans, guar gum, locust beam gum. One suitable edible matrix comprises a gel prepared from concentrated Aloe Vera extract: a smooth creamy paste (suitable for packaging in a squeezable tube) may be prepared by mixing 5gms of creatine with 20mls of a concentrated Aloe Vera gel (such as that obtainable from Aloe Commodities Int. Inc., Farmers Branch, TX75234).

Preferably the bar will have a chewy texture, to discourage rapid ingestion. The desirability of prolonging the interval during which the bar is ingested is discussed further below.

The inventors have previously found that, by providing creatine in the form of a solid in suspension, rather than in solution, conversion to creatinine (even in an acidic composition) can be greatly inhibited or even substantially prevented even at ambient (i.e. 20-25°C) temperature (WO 00/74500). Thus, in some embodiments the composition as a whole (and/or the supporting matrix in isolation) may conveniently be selected to be any pH, acidic (i.e. have a pH below 7.0) or alkaline (pH 7.0-8.5), without significantly adversely affecting the stability of the creatine content of the composition. In particular the composition desirably has a pH between 3.0 and 8.5, preferably between 3.5 and 8.0. In some embodiments the composition may have a pH in the range 3.5-5.5 which, to the human palate, has a refreshingly sharp taste without being too acidic.

Snack bars in accordance with the invention are substantially stable so that creatine may be presented in acidic formulations, contrary to the teaching of the art, in physiologically useful amounts, even following storage for prolonged periods at ambient temperature. A physiologically useful amount of creatine is an amount sufficient to cause a measurable increase in the creatine content of the tissues of a subject following repeated consumption of the composition, relative to an initial baseline level.

The term "substantially stable" is herein defined as referring to a composition in which at least 85 % of the original creatine in the composition is unchanged into creatinine for a period of at least 7 days' storage at ambient (20-25°C) temperature. Desirably the composition will be sufficiently stable that 85 % of the creatine remains following a period of at least 31 days', more preferably 45 days' , and most preferably at least 73 days' storage at ambient temperature.

The amount of creatine per Kg of prepared snack bar may range from 0.5 to 200g, with a preferred content of about 100g per Kg. The normal serving size is in the range 25-250g, providing about 2-5g (preferably about 3g) of creatine. During the first 4-5 days of creatine supplementation the recommended consumption is about 25g/day, to achieve creatine saturation. This is followed by about 3-5g of creatine per day (usually consumed in a single serving) to provide a maintenance level of creatine.

Each bar will typically have a mass in the range 15-250g, preferably about 30-75g. The bars may be individually wrapped, or a plurality of bars may be collectively packaged and wrapped. Suitable packaging and wrapping will be apparent to those skilled in the art. Preferably the wrapping will comprise a layer of moisture-impermeable material, such as a metallised foil or a synthetic plastics material.

Preferably the bar may comprise a coating, which improves one or more of the appearance, flavor and texture of the bar. Examples of coatings include chocolate-flavored coatings (white or dark chocolate) and yoghurt.

Previous work by others has shown that creatine saturation of muscle in humans can be achieved by consumption of a 'loading' dose of creatine in the range 20-35g/day creatine over 4-5 days and thereafter creatine saturation can be maintained by consumption of a lower 'maintenance' dose of 2-10g (preferably 3-5g) creatine per day.

The creatine content of a snack bar in accordance with the invention will conveniently be such as to provide a maintenance dose of creatine in a single bar of about 15-75g in mass, and a loading dose of creatine provided by consumption of several such bars or by consumption of a smaller number of larger bars (e.g. a 75-150g size). Typically the creatine content of the bar will be 0.25g-15g, more preferably 3-5g for a bar of 15-75g, and 3-10g of creatine for a bar of 75-150g size.

It is preferred that the overall water content of the bar is low (i. e. less than 20 % w/w, preferably less than 15%, more preferably less than 10%), such that the creatine content of the bar substantially remains in solid form and does not become dissolved, thereby preventing the conversion of creatine to the inactive compound creatinine. Alternatively, if the overall water content of the bar is greater than 20%, it is preferred that the creatine is provided in a discrete portion or layer of the bar in which the water content is less than 20%, and wherein ingress of moisture into said portion or layer is substantially prevented (e.g. by provision of an edible, water-impermeable coating, such as a yogurt coating).

The bars may be eaten as a snack, or a large bar or plurality of smaller bars eaten as a meal replacement (preferably with a beverage such as water, tea, coffee etc).

The bars may be formed by any convenient conventional method e.g. cutting and/or extruding.

One typical embodiment of the invention takes the form of a snack bar comprising a cereal, solid creatine, low glycemic index sweetener, one or more flavorings, raising agent and margarine. The ingredients are mixed, briefly baked, cooled and then cut into bars. An alternative embodiment (suitable for manufacture by extrusion) takes the form of a smooth, semi-solid mixture comprising protein (e.g. soya isolate, whey protein, caseinate, or other milk- or soya- derived proteinaceous product), solid creatine, a sweetener and glycerin or a syrup.

In a second aspect, the invention provides a method of making a creatine-containing low glycemic index snack bar for human consumption, the method comprising the steps of: mixing solid creatine with an edible supporting matrix, and optionally one or more further ingredients, such that the glycemic index of the mixture as a whole has a value of 55 or below; forming the resulting mixture into a bar; and, optionally, packaging the bar.

Typically, the solid creatine will be mixed with other solid components before being mixed with the edible supporting matrix. A preferred edible supporting matrix comprises butter, margarine or other fat which is conveniently melted to facilitate mixing with the creatine and other solid ingredients which may optionally be present. Preferably the edible supporting matrix also functions as a binding agent, serving to bind the ingredients together.

The mixed ingredients may be subjected to further processing steps e.g. baking, cooling etc. The preferred manner in which the mixed ingredients are formed into a bar may depend, at least in part, on the physical properties of the composition. Thus, for instance, some compositions may be formed into bars by extrusion through a die of suitable size and shape, and then cutting the extruded composition into desired lengths. Alternatively the composition may be allowed to harden in moulds, or may be baked and cut.

Once formed, the bars may be packaged in any desired manner (e.g. singly or in packets). Preferably the packaging will comprise the step of providing a moisture impermeable wrapping (e.g. a metallic foil or a synthetic plastics material) around a bar or plurality of bars. Such methods are well known to those skilled in the art.

It is predicted that consumption of a snack bar in accordance with the invention will have a marked physiologically beneficial effect on subjects suffering from hyperglycaemia and related conditions, such as diabetes.

The inventors believe that the beneficial effect will arise from a synergistic combination of
(i) consumption of creatine;
(ii) consumption of a low glycemic index foodstuff; and
(iii) creatine release into the bloodstream from the gut being sustained over a prolonged period (relative to consumption of a creatine tablet or drink) due to its incorporation into a snack bar. It is physically impossible to consume very rapidly a snack bar, especially if it has a chewy texture, so the snack bar will be ingested over a prolonged period. Secondly, the incorporation of the creatine within the matrix of the bar results in a steady, sustained release of creatine as the matrix is digested. Such sustained release is expected to result in improved pharmacodynamics and pharmacokinetics, and cause depression of blood glucose levels over a longer period than consumption of an equivalent dose of creatine as a bolus in a drink, tablet, capsule or the like.

The release of creatine from an ingested bar can be further sustained by incorporating creatine in the bar in a slow-release or sustained-release form. For example, the creatine can be encapsulated in lipid-based or protein-based capsules, using conventional encapsulation techniques (see, for example, WO 01/35953).

The blood glucose level can be readily measured using any of a number of conventional tests.

A "hyperglycemic condition" includes, for present purposes, hyperglycemia, insulin insensitivity, diabetes mellitus, and the like.

One of the most popular methods of weight reduction is the so-called "Low Glycemic Index Diet" which has been popularised by such publications such as "The Low GI Diet. Lose Weight with Smart Carbs (by J Brand-Miller *et al* Hodder & Stoughton, London, 2004) and "The GI Diet" (by Rick Gallop, Virgin, London, 2003, among others) which can be summed up as follows:
low GI foods are slower to digest, so a dieter will feel satisfied longer;
low GI foods keep insulin levels low thereby inhibiting the formation of fat, and assisting in the conversion of fat back to energy; and
the key to losing weight is to eat low GI and low calorie foods.

The inventors believe that a low glycemic index snack bar, particularly as a meal replacement or part of a low calorie meal and/or low glycemic index meal, is a helpful aid to those wishing to lose weight by the above-mentioned GI method. Subjects on a low calorie diet invariably complain of fatigue. The inclusion of creatine in the low glycemic bar is beneficial in preventing or reducing this symptom. Thus a low glycemic index bar containing creatine is a useful adjunct to a calorie-controlled diet.

The present invention is demonstrated by the following non-limiting examples.

### Example 1

An oat based cooked bar with high energy content, but low glycemic index, containing at least 5grams creatine monohydrate per bar.

### Ingredients (per 100g)

| | |
|---|---|
| Oats | 40g |
| Fructose syrup (circa 90 % fructose) | 15g |
| Honey | 15g |
| Margarine | 23g |
| Creatine monohydrate (micronised) | 5.5g |
| Raising agent (e.g. sodium bicarbonate; disodium phosphate) | 0.5g |
| Flavoring, and preservative (potassium sorbate) | 1g |

### Nutritional profile

| | |
|---|---|
| Energy | 415 kcal |
| Protein | 5g |
| Carbohydrate | 57g |
| Fat | 19g |
| Fibre | 4g |

### Optional Additional Flavorings

### (i) Cherry Flavor

Add: glacé cherries 6%

### (ii) Banana

Add: dried bananas (24%), wholemeal flour, sugar, hazelnuts

### Optional Additional coatings

### (iii) White chocolate flavor

Add: white chocolate flavour coating 10% (sugar, hydrogenated vegetable oil, whey powder, wheat flour, palm oil, emulsifier, flavoring).

### (iv) Chocolate Flavor

Add: chocolate flavor coating 10 % (sugar, hydrogenated vegetable oil, whey powder, milk solids, lactose, palm oil, fat reduced cocoa powder, emulsifiers, flavoring).

### (v) Yoghurt Flavor

Add: yoghurt flavour coating 10% (sugar, hydrogenated vegetable oil, yoghurt powder, whey powder, wheat flour, palm oil, emulsifier, flavoring).

### Method of manufacture

The margarine is melted by gentle heating and added to the syrup and well mixed in a suitable commercial mixer. The dry ingredients (oats, creatine monohydrate, raising agent) are mixed separately and then added to the liquid and well mixed. The whole mix is placed in a tray about 1 inch deep and placed in a hot oven at 250°C for 6 min. It is then allowed to cool to room temperature and cut into 100g pieces. These are then wrapped in foil.

### Additional flavorings

For the cherry and banana flavors the appropriate amounts of additional ingredients are added to the basic mix and the amount of creatine monohydrate is adjusted pro-rata to give a final concentration of 5 % w/w.

### Coatings

The appearance of the bar is vastly improved by coating it with a desirable edible coating such as chocolate or yoghurt. This is applied after the cooked mass is cooled and cut up into bars. The melted coating is applied to each bar to increase its weight by about 10% and the bar is placed in a cooling tunnel before wrapping.

### Creatine content

After manufacture a sample of bars without additional flavoring and uncoated were analysed for creatine content. The loss of creatine during processing was negligible (see example 4). During subsequent weeks the creatine content remained remarkably stable at room temperature.

### Example 2

An extruded chocolate-flavored bar of low glycemic index containing about 3g creatine.

### Ingredients (per bar)

| | |
|---|---|
| Protein Blend (Whey Protein Isolate, Soy Protein Isolate, Calcium Caseinate), | 20g |
| Fructose Powder | 10g |
| Sorbitol | 10g |
| Glycerol | 5.5g |
| Creatine monohydrate 3g | |
| Glucose syrup, Cocoa Liquor, Cocoa Powder, Natural & Artificial Flavors, | 1g |
| Soya Lecithin | 1g |
| Water | 1g |
| Sucralose | 0.25g |

Chocolate Coating (10% mass of bar): maltitol, partially fractionated Palm kernel oil, whey protein concentrate, cocoa powder, calcium carbonate, natural flavor, soya lecithin sucralose.

### Nutritional profile (per bar)

| | |
|---|---|
| Energy | 160 kcal. |
| Protein | 15g |
| Carbohydrate | 25g |
| Fat | 5g |

### Manufacture

The dry ingredients and the liquid ingredients are mixed separately and then added together with further mixing. The whole is then extruded at room temperature and cut into bars. The chocolate coating is heated and applied, the bars cooled and wrapped.

### Example 3

Extruded chocolate bar containing at least 3g creatine, with a low glycemic index, containing vitamins and minerals.

### Ingredients (per bar)

| | |
|---|---|
| Protein Blend (whey protein isolate, soy protein isolate, calcium caseinate) | 20g |
| Maltitol | 20g |
| Glycerol | 5g |
| Creatine monohydrate | 3.3g |
| Brown Rice Syrup | 2g |
| Cocoa Liquor, Cocoa Powder, Natural & Artificial Flavors | 1g |
| Soya Lecithin | 1g |
| Water | 1g |
| Sucralose | 0.7g |
| Vitamin mix* (containing 10% RDA) | 1g |

| | |
|---|---|
| * Vitamins and Minerals included in mix : dicalcium phosphate, dipotassium phosphate, magnesium oxide, monocalcium phosphate, ascorbic acid, ferric orthophosphate, vitamin E acetate, niacinamide, zinc oxide, copper gluconate, d-calcium pantothenate, manganese sulfate, vitamin A palmitate, pyridoxine hydrochloride, thiamin mononitrate, riboflavin, sodium molybdate, chromium chloride, folic acid, biotin, sodium selenite, potassium iodide, vitamin K1 (Phytonadione), vitamin B12. | |

### Chocolate coating (10% of weight)

Maltitol, partially fractionated palm kernel oil, whey protein concentrate, cocoa powder, calcium carbonate, natural flavor, soya lecithin, sucralose.

### Nutritional Composition (per bar)

| | |
|---|---|
| Energy | 160 kcal |
| Protein | 15 g |
| Carbohydrate | 25g |
| Fat | 5g |

### Manufacture

The dry ingredients and the liquid ingredients are mixed separately and then added together with further mixing. The whole is then extruded at room temperature and cut into bars. The chocolate coating is heated and applied, the bars cooled and wrapped.

### Creatine content

Analysis of creatine showed a negligible loss of creatine during manufacture (see Example 4). No further loss of creatine occurred even after several weeks at room temperature.

### Example 4

Analysis of creatine in manufactured bars

### A) Method of analysis

1. Bars were weighed to three decimal places and placed in a blender with 400ml warm distilled water (dH₂O) and homogenised during 3 x 30s bursts. The homogenate and washings (prepared using cold dH₂O) from the blender were poured into a volumetric flask, made up to 2 litres and mixed over 5 min. A 1ml sample of the final homogenate was clarified by centrifugation at 10,000rpm and 250µl was added to approximately 3ml of dH₂O (exact volume determined by weighing to 4 decimal places).
2. Creatine was measured photometrically at 340nm by means of a linked enzymic assay, in 25µl of diluted extract in a final assay volume of 275µl using a microplate reader. Assays were conducted in the presence of lactate dehydrogenase, pyruvate kinase, 30mM K⁺, 10mM Mg²⁺, 1mM EDTA, 1mM phosphoenolpyruvate, 2mM adenosine triphosphate, 0.3mM nicotinamide dinucleotide. Creatine kinase was added to initiate the assay. Changes in absorbance were compared to those obtained with standards of creatine monohydrate of 0.5 to 2mM
3. Creatinine was assayed at 500nm using the alkaline picrate method and the kit (555-A) supplied by Sigma Diagnostics, in 30µl of diluted extract in a final volume of 280µl. The difference in absorbance between samples and blanks were compared to those obtained with standards of creatinine of 0.5 to 2mM.

### B) Results

Creatine-containing bars according to Example 1, or Example 3, were analysed for their creatine content, before and after storage. The results are presented in the Table below.

| Type of Bar | Flavor | Cr.H₂O Added per 50g bar | Wt of bar | Cr.H₂O Per bar By Analysis | Creatinine per bar by analysis | Cr.H₂O per 50g by analysis | Cr.H₂O* per 50g by calculation |
|---|---|---|---|---|---|---|---|
| | | g | g | G | g | g | g |
| 5g Cr Example 1 pH 7.18 | Plain | 5.0 | 60.21 (58.49**) | 6.087 (6.069**) | 0.062 (0.035**) | 5.055 (5.188**) | 5.122 (5.228**) |
| 5g Cr Comparative Example 1 pH 7.18 | Plain | 5.0 | 58.41 | 5.959 | 0.050 | 5.101 | 5.158 |
| 5g Cr Comparative Example 1 pH7.18 | Plain | 5.0 | 61.90 | 6.416 | 0.073 | 5.183 | 5.260 |
| 3g Cr Comparative Example 1 pH 7.12 | Plain | 3.0 | 61.14 (60.96**) | 3.900 (3.924**) | 0.070 (0.087**) | 3.189 (3.219**) | 3.265 (3.313**) |
| Example 3 | Peanut 5.0 | | 48.00 | 4.486 | 0.053 | 4.673 | 4.746 |
| Example 3 pH 7.29 | Chocolate | 5.0 | 49.01 (49.01**) | 4.972 (4.846**) | 0.063 (0.112**) | 5.073 (4.943**) | 5.158 (5.094**) |
| Example 3 | Chocolate | 5.0 | 46.99 | 5.083 | 0.079 | 5.408 | 5.519 |

| | | | | | | | |
|---|---|---|---|---|---|---|---|
| *Sum of creatine by analysis + creatine converted to creatinine ** Second sample of bar analysed 4 weeks after the first analysis | | | | | | | |

The bars analysed in the above table described as "comparative Example 1" contained partially inverted refined syrup, instead of fructose syrup and honey, and did not have a low glycemic index.

Bars according to Example 1 were first analysed 4 weeks after manufacture, whilst bars according to Example 3 were first analysed 2 weeks after manufacture. All bars were stored at room temperature prior to analysis.

### C) Conclusions

The results show that very little creatine was destroyed during the manufacture of the bars since the analysis of creatine present is similar to the amount added. Also the amount of creatinine from creatine formed is very small.

### Example 5

Preparation of encapsulated creatine for sustained release formulation.

Creatine monohydrate is added to a vertical high intensity mixer which can be operated and kept at relatively high temperatures e.g. 90°C. A hydrogenated oil, such as hydrogenated soy oil (melting point circa 80° C), is melted and sprayed slowly on to the powder kept at 90°C while the mixer is in operation. The total amount of oil added is about 25% of the weight of the creatine monohydrate. After a further 5 min. mixing the oil coated powder is cooled to produce free flowing granules with sustained release properties.

The granules are suitable for inclusion in a snack bar such as described in examples 1-3 as a source of creatine monohydrate, care being taken that the temperature of mixing the ingredients in the bar is kept below the melting point of the hydrogenated oil used above.

### Example 6

Test for efficacy of a low glycemic index creatine-containing snack bar in treating and/or preventing hyperglycemia or diabetes.

### 1) Effect of creatine alone

It is important to establish if creatine alone has any significant effect in diabetics. Diabetics on the drug Metformin (it is virtually impossible to recruit untreated patients) will be taken off the drug for 2 weeks prior to the trial and during the trial.

They will be divided into 2 groups (with a minimum of 5 patients per group) and given either:
(A) 20g creatine per day (in divided doses of 5g every 3 hours) for 5 days (in order to saturate the body with creatine); or
(B) a placebo for the same period.

An oral glucose tolerance test (OGTT) in fasting patients will be conducted before and after treatment. This consists of giving 75g glucose in water and taking blood before dosing and every 30 minutes for 150 minutes thereafter and measuring blood glucose. The area under the blood glucose versus time curve is measured.

If creatine alone is effective in reducing blood glucose levels the change in the area under the blood glucose versus time curve will be smaller in the creatine group (A) than that in the placebo (B).

### 2) Effect of creatine in a low glycemic index bar

This is a much longer trial since it is intended to give only 5g creatine per day. As before diabetic patients would be taken off Metformin for 2 weeks prior to commencing the trial and then divided into 4 groups (with a minimum of 5 subjects per group) and given at lunchtime one of the following:
a) a low glycemic index bar containing 5g creatine daily for 4 weeks (during which time the body would be saturated with creatine);
b) the same low glycemic index bar without creatine;
c) a dose of 5g creatine powder in water; or
d) a dose of placebo powder in water.

An OGTT will be conducted before and after treatment as above.

In addition glycosylated haemaglobin (HbA1C) and fructosamine (FA) in the blood will be measured before and after treatment. HbA1C and FA are indices of the level of blood glucose over a prolonged period.

If creatine is effective
i) the area under the OGTT after treatment minus before treatment in (a) will be less than in (b). Likewise for (c) versus (d).
ii) The difference between HbA1C and FA values before and after treatment in (a) will be less than (b). Likewise for (c) versus (d).

If the low glycemic index bar is effective the above mentioned difference values will be greater in (b) than in (d).

If there is a synergistic effect of the creatine and the bar combined, the effects of creatine must apply as follows:

The effect of (creatine in the bar minus the effect of the bar only) must be greater than the sum of (the effect of the bar minus the effect of water only) plus (the effect of creatine in water minus the effect of water only). That is, (a)-(b) must be greater than the sum of (( (b)-(d)) + ((c)-(d)).

## Claims

1. A solid or semi-solid snack bar for human consumption, comprising creatine monohydrate suspended in solid form in an edible supporting matrix; the food bar as a whole having a glycemic index of less than 55.

2. A snack bar according to claim 1, comprising one or more additional ingredients selected from the group consisting of fruit; cereals; nuts; cocoa; chocolate; vitamins; minerals; lipids; carbohydrates; fibre, flavors; artificial sweeteners; and preservatives.

3. A snack bar according to claim 1 or 2, comprising one or more protein concentrates

4. A snack bar according to claim 3, wherein the protein concentrate is selected from a group comprising soy protein, whey protein and caseinates.

5. A snack bar according to any one of the preceding claims, wherein the edible supporting matrix comprises a spreadable solid.

6. A snack bar according to claim 5, wherein the spreadable solid comprises butter, margarine or other fat.

7. A snack bar according to any one of the preceding claims, comprising a natural sweetener.

8. A snack bar according to claim 7, wherein the natural sweetener is selected from the group consisting of: fructose syrup or high fructose containing glycerol; sorbitol; and maltitol.

9. A snack bar according to any one of the preceding claims, comprising creatine monohydrate in encapsulated or other sustained-release form

10. A snack bar according to any one of the preceding claims, comprising an artificial sweetener.

11. A snack bar according to claim 10, wherein the artificial sweetener is selected from the group consisting of: aspartame; acesulfame K; saccharine; and sucralose.

12. A snack bar according to any one of the preceding claims, comprising one or more vitamins and minerals selected from the group consisting of: dicalcium phosphate, dipotassium phosphate, magnesium oxide, monocalcium phosphate, ascorbic acid, ferric orthophosphate, vitamin E acetate, niacinamide, zinc oxide, copper gluconate, d-calcium pantothenate, manganese sulfate, vitamin A palpitate, pyridoxine hydrochloride, thiamin mononitrate, riboflavin, sodium molybdate, chromium chloride, folic acid, biotin, sodium selenite, potassium iodide, vitamin K1 (phytonadione), and vitamin B 12.

13. A snack bar according to any one of the preceding claims, comprising at least 0.25 g creatine per bar.

14. A snack bar according to claim 13, comprising at least 2g creatine per bar.

15. A snack bar according to claim 13, comprising at least 5g creatine per bar.

16. A snack bar according to any one of the preceding claims, having a mass in the range 15-250g.

17. A snack bar according to claim 16, having a mass in the range 30-150g.

18. A snack bar according to any one of the preceding claims, comprising an edible coating.

19. A snack bar according to claim 18, wherein the coating comprises a chocolate or chocolate-flavored coating or a yogurt coating.

20. A method of making a creatine-containing low glycemic index snack bar for human consumption, the method comprising the steps of: mixing solid creatine monohydrate with an edible supporting matrix, and optionally one or more further ingredients, such that the glycemic index of the mixture as a whole has a value of 55 or below; forming the resulting mixture into a bar; and packaging the bar.

21. A method according to claim 20, wherein all solid ingredients are mixed together prior to mixing with the edible supporting matrix.

22. A method according to claim 20, wherein the edible supporting matrix comprises butter, margarine or other fat.

23. A method according to claim 20, wherein forming the mixture into a bar comprises the step of extruding and/or cutting the mixture.

## Patentansprüche

1. Fester oder halbfester Snackriegel für den menschlichen Verzehr, umfassend Kreatinmonohydrat, das in fester Form in einer verzehrbaren Trägermatrix suspendiert ist; wobei der Nahrungsmittelriegel als Ganzes einen glykämischen Index von weniger als 55 aufweist.

2. Snackriegel gemäß Anspruch 1, umfassend einen oder mehrere zusätzliche Inhaltsstoffe, ausgewählt aus der Gruppe, bestehend aus: Früchten; Getreide; Nüssen; Kakao; Schokolade; Vitaminen; Mineralstoffen; Lipiden; Kohlenhydraten; Fasern; Geschmacksstoffen; künstlichen Süßungsmitteln; und Konservierungsmitteln.

3. Snackriegel gemäß Anspruch 1 oder 2, umfassend ein oder mehrere Proteinkonzentrate.

4. Snackriegel gemäß Anspruch 3, wobei das Proteinkonzentrat ausgewählt ist aus der Gruppe, umfassend Sojaprotein, Molkeprotein und Caseinate.

5. Snackriegel gemäß einem der vorstehenden Ansprüche, wobei die verzehrbare Trägermatrix einen streichbaren Feststoff umfasst.

6. Snackriegel gemäß Anspruch 5, wobei der streichbare Feststoff Butter, Margarine oder ein anderes Fett umfasst.

7. Snackriegel gemäß einem der vorstehenden Ansprüche, umfassend ein natürliches Süßungsmittel.

8. Snackriegel gemäß Anspruch 7, wobei das natürliche Süßungsmittel ausgewählt ist aus der Gruppe, bestehend aus: Fructosesirup oder Glycerol mit hohem Fructosegehalt; Sorbitol; und Maltitol.

9. Snackriegel gemäß einem der vorstehenden Ansprüche, umfassend Kreatinmonohydrat in verkapselter Form oder einer anderen Form mit anhaltender Freisetzung.

10. Snackriegel gemäß einem der vorstehenden Ansprüche, umfassend ein künstliches Süßungsmittel.

11. Snackriegel gemäß Anspruch 10, wobei das künstliche Süßungsmittel ausgewählt ist aus der Gruppe, bestehend aus: Aspartam; Acesulfam K; Saccharin; und Sucralose.

12. Snackriegel gemäß einem der vorstehenden Ansprüche, umfassend ein oder mehrere Vitamine und Mineralstoffe, ausgewählt aus der Gruppe, bestehend aus: Dicalciumphosphat, Dikaliumphosphat, Magnesiumoxid, Monocalciumphosphat, Ascorbinsäure, Eisen(III)-orthophosphat, Vitamin-E-acetat, Niacinamid, Zinkoxid, Kupfergluconat, d-Calciumpantothenat, Mangansulfat, Vitamin-A-palmitat, Pyridoxinhydrochlorid, Thiaminmononitrat, Riboflavin, Natriummolybdat, Chromchlorid, Folsäure, Biotin, Natriumselenit, Kaliumiodid, Vitamin K1 (Phytonadion) und Vitamin B12.

13. Snackriegel gemäß einem der vorstehenden Ansprüche, umfassend wenigstens 0,25 g Kreatin pro Riegel.

14. Snackriegel gemäß Anspruch 13, umfassend wenigstens 2 g Kreatin pro Riegel.

15. Snackriegel gemäß Anspruch 13, umfassend wenigstens 5 g Kreatin pro Riegel.

16. Snackriegel gemäß einem der vorstehenden Ansprüche mit einer Masse im Bereich von 15-250 g.

17. Snackriegel gemäß Anspruch 16 mit einer Masse im Bereich von 30-150 g.

18. Snackriegel gemäß einem der vorstehenden Ansprüche, umfassend einen verzehrbaren Überzug.

19. Snackriegel gemäß Anspruch 18, wobei der Überzug Schokolade oder einen Überzug mit Schokoladegeschmack oder einen Joghurtüberzug umfasst.

20. Verfahren zum Herstellen eines Kreatinenthaltenden Snackriegels für den menschlichen Verzehr mit einem niedrigen glykämischen Index, wobei das Verfahren die Schritte umfasst: Mischen von festem Kreatinmonohydrat mit einer verzehrbaren Trägermatrix und gegebenenfalls einem oder mehreren weiteren Inhaltsstoffen, so dass der glykämische Index des Gemischs als Ganzes einen Wert von 55 oder weniger aufweist; Formen des so erhaltenen Gemischs zu einem Riegel; und Verpacken des Riegels.

21. Verfahren gemäß Anspruch 20, wobei alle festen Inhaltsstoffe vor dem Mischen mit der verzehrbaren Trägermatrix zusammengemischt werden.

22. Verfahren gemäß Anspruch 20, wobei die verzehrbare Trägermatrix Butter, Margarine oder ein anderes Fett umfasst.

23. Verfahren gemäß Anspruch 20, wobei das Formen des Gemischs zu einem Riegel den Schritt des Extrudierens und/oder Schneidens des Gemischs umfasst.

## Revendications

1. Barre encas solide ou semi-solide destinée à la consommation humaine, comprenant de la créatine monohydratée mise en suspension sous forme solide dans une matrice support comestible ; l'ensemble de la barre alimentaire ayant un index glycémique inférieur à 55.

2. Barre encas selon la revendication 1, comprenant un ou plusieurs ingrédients supplémentaires choisis dans le groupe constitué de : fruit ; céréales ; noix ; cacao ; chocolat ; vitamines ; minéraux ; lipides ; glucides ; fibres, arômes ; édulcorants artificiels ; et agents conservateurs.

3. Barre encas selon la revendication 1 ou 2, comprenant un ou plusieurs concentrés protéiques.

4. Barre encas selon la revendication 3, **caractérisée en ce que** le concentré protéique est choisi dans un groupe comprenant de la protéine de soja, de la protéine de lactosérum et des caséinates.

5. Barre encas selon l'une quelconque des revendications précédentes, **caractérisée en ce que** la matrice support comestible comprend un solide tartinable.

6. Barre encas selon la revendication 5, **caractérisée en ce que** le solide tartinable comprend le beurre, la margarine ou d'autres graisses.

7. Barre encas selon l'une quelconque des revendications précédentes, comprenant un édulcorant naturel.

8. Barre encas selon la revendication 7, **caractérisée en ce que** l'édulcorant naturel est choisi dans le groupe constitué de : sirop de fructose ou glycérol riche en fructose ; sorbitol ; et maltitol.

9. Barre encas selon l'une quelconque des revendications précédentes, comprenant de la créatine monohydratée sous forme encapsulée ou sous une autre forme à libération prolongée.

10. Barre encas selon l'une quelconque des revendications précédentes, comprenant un édulcorant artificiel.

11. Barre encas selon la revendication 10, **caractérisée en ce que** l'édulcorant artificiel est choisi dans le groupe constitué de : aspartame ; acésulfame K ; saccharine ; et sucralose.

12. Barre encas selon l'une quelconque des revendications précédentes, comprenant un ou plusieurs vitamines et minéraux choisis dans le groupe constitué de : phosphate dicalcique, phosphate dipotassique, oxyde de magnésium, phosphate monocalcique, acide ascorbique, orthophosphate ferrique, acétate de vitamine E, niacinamide, oxyde de zinc, gluconate de cuivre, d-pantothénate de calcium, sulfate de manganèse, palmitate de vitamine A, chlorhydrate de pyridoxine, mononitrate de thiamine, riboflavine, molybdate de sodium, chlorure de chrome, acide folique, biotine, sélénite de sodium, iodure de potassium, vitamine K1 (phytonadione), et vitamine B12.

13. Barre encas selon l'une quelconque des revendications précédentes, comprenant au moins 0,25 g de créatine par barre.

14. Barre encas selon la revendication 13, comprenant au moins 2 g de créatine par barre.

15. Barre encas selon la revendication 13, comprenant au moins 5 g de créatine par barre.

16. Barre encas selon l'une quelconque des revendications précédentes, ayant une masse dans la gamme de 15-250 g.

17. Barre encas selon la revendication 16, ayant une masse dans la gamme de 30-150 g.

18. Barre encas selon l'une quelconque des revendications précédentes, comprenant un enrobage comestible.

19. Barre encas selon la revendication 18, **caractérisée en ce que** l'enrobage comprend un enrobage au chocolat ou chocolaté ou un enrobage ou yaourt.

20. Méthode de fabrication d'une barre encas de faible index glycémique contenant de la créatine destinée à la consommation humaine, la méthode comprenant les étapes consistant à : mélanger de la créatine solide monohydratée avec une matrice support comestible, et éventuellement un ou plusieurs autres ingrédients, de sorte que l'index glycémique de l'ensemble du mélange ait une valeur de 55 ou moins ; former le mélange résultant en une barre ; et emballer la barre.

21. Méthode selon la revendication 20, **caractérisée en ce que** tous les ingrédients solides sont mélangés ensemble avant le mélange avec la matrice support comestible.

22. Méthode selon la revendication 20, **caractérisée en ce que** la matrice support comestible comprend du beurre, de la margarine ou d'autres graisses.

23. Méthode selon la revendication 20, **caractérisée en ce que** la formation du mélange en une barre comprend l'étape d'extrusion et/ou de découpage du mélange.
